# FASCICULE DE BREVET EUROPEEN

(11) **EP 2 349 960 B1**
(45) Date de publication et mention de la délivrance du brevet: **29.04.2015**
(21) Numéro de dépôt: 09740471.9
(22) Date de dépôt: 19.08.2009
(51) Int. Cl.: C07C 17/25, C07C 17/354, C07C 21/18, C07C 19/08

(54) **PROCEDE DE PREPARATION DE COMPOSES FLUORES**
VERFAHREN ZUR HERSTELLUNG FLUORIERTER VERBINDUNGEN
PROCESS FOR THE PREPARATION OF FLUORINATED COMPOUNDS

(30) Priorité: 11.09.2008 FR 0856114
(43) Date de publication de la demande: 03.08.2011
(73) Titulaire: ARKEMA FRANCE, 92700 Colombes (FR)
(72) Inventeur: PIGAMO, Anne, F-69340 Francheville (FR); DEVIC, Michel, F-69110 Sainte Foy Les Lyon (FR); WENDLINGER, Laurent, F-69510 Soucieu En Jarrest (FR)
(74) Mandataire: Dang, Doris
(86) Numéro de dépôt international: PCT/FR2009/051600
(87) Numéro de publication internationale: WO 2010/029240

(56) Documents cités:
- US-A1- 2007 179 324
- SIANESI DARIO ET AL: "Fluoro olefins. I. cis- and trans-1,2,3,3,3-Pentafluoropropylene" ANNALI DI CHIMICA, SOCIETA CHIMICA ITALIANA, ROME, IT, vol. 55, no. 8-9, 1 janvier 1965 (1965-01-01), pages 850-861, XP009092725 ISSN: 0003-4592 cité dans la demande
- KNUNYANTS I L ET AL: "REACTIONS OF FLUORO OLEFINS. ÖCOMMUNICATION 13. CATALYTIC HYDROGENATION OF PERFLUORO OLEFINS", BULLETIN OF THE ACADEMY OF SCIENCES OF THE USSR, DIVISION OF CHEMICAL SCIENCES, SPRINGER NEW YORK LLC, US, 1 January 1960 (1960-01-01), pages 1312-1317, XP000578879, ISSN: 0568-5230, DOI: 10.1007/BF00907661

## Description

### DOMAINE DE L'INVENTION

L'invention a pour objet un procédé de préparation de composés fluorés, à savoir le composé fluoré 2,3,3,3-tétrafluoro-1-propène.

### ARRIERE-PLAN TECHNOLOGIQUE

Les hydrofluorocarbones (HFC) et en particulier les hydrofluorooléfines, telles que le 2,3,3,3-tétrafluoro-1-propène (HFO 1234yf) sont des composés connus pour leurs propriétés de réfrigérants et fluides caloporteurs, extinctrices, propulseurs, agents moussants, agents gonflants, diélectriques gazeux, milieu de polymérisation ou monomère, fluides supports, agents pour abrasifs, agents de séchage et fluides pour unité de production d'énergie. A la différence des CFC et des HCFC, qui sont potentiellement dangereux pour la couche d'ozone, les HFOs ne contiennent pas de chlore et donc ne posent pas de problème pour la couche d'ozone.

On connaît plusieurs procédés de fabrication du 1234yf.

WO2008/002499 décrit un procédé de production d'un mélange de 2,3,3,3-tétrafluoro-1-propène (HFO-1234yf) et de 1,3,3,3-tétrafluoro-1-propène (HFO-1234ze) par pyrolyse de 1,1,1,2,3-pentafluoropropane (HFC-245eb).

WO2008/002500 décrit un procédé de production d'un mélange de 2,3,3,3-tétrafluoro-1-propène (HFO-1234yf) et de 1,3,3,3-tétrafluoro-1-propène (HFO-1234ze) par conversion catalytique de 1,1,1,2,3-pentafluoropropane (HFC- 245eb) sur un catalyseur de déhydrofluoration.

Ces deux demandes précitées visent donc la production d'un mélange contenant une partie substantielle de produit HFO-1234ze.

WO2007/056194 décrit la préparation de HFO-1234yf par déhydrofluoration de HFC-245eb, soit avec de la potasse, typiquement une solution aqueuse d'au plus 50 % en poids de KOH, soit en phase gazeuse en présence d'un catalyseur, notamment catalyseur à base de nickel, de carbone ou une combinaison de ceux-ci.

Le document Knunyants et al, Journal of the USSR Academy of Sciences, Chemistry Department, "reactions of fluoro-olefins", report 13., "catalytic hydrogenation of perfluoro-olefins", 1960, décrit de façon distincte diverses réactions chimiques sur des composes fluorés. Ce document décrit l'hydrogénation sensiblement quantitative de HFP sur un catalyseur à base de palladium supporté sur alumine, la température passant de 20°C à 50°C, puis étant maintenue à cette valeur. Ce document décrit la déhydrofluoration du 1,1,1,2,3,3-hexafluoropropane (HFC-236ea) par passage au travers d'une suspension de KOH dans l'éther de dibutyle, pour produire du 1,2,3,3,3-pentafluoropropène-1 (HFO-1225ye) avec un rendement de 60 % seulement. Ce document décrit l'hydrogénation du 1,2,3,3,3-pentafluoropropène-1 (HFO-1225ye) en 1,1,1,2,3-pentafluoropropane (HFC-245eb) sur un catalyseur palladium supporté sur alumine. Au cours de cette hydrogénation il se produit aussi une réaction d'hydrogénolyse, une quantité significative de 1,1,1,2-tetrafluoropropane étant produite. Ce document décrit la déhydrofluoration de 1,1,1,2,3-pentafluoropropane (HFC-245eb) en 2,3,3,3-tétrafluoro-1-propène (HFO-1234yf) par passage dans une suspension de KOH en poudre dans l'éther de dibutyle avec un rendement de 70 % seulement. Ces réactions sont décrites indépendamment les unes des autres, mêmes s'il est indiqué qu'il est possible de les combiner pour synthétiser une gamme de dérivés d'éthylène, propylène et isobutylène contenant des quantités variables de fluor.

Le document US-P-5396000 décrit la préparation de 1,1,1,2,3-pentafluoropropane par déhydrofluoration catalytique de 1,1,1,2,3,3-hexafluoropropane (HFC-236ea) en 1,2,3,3,3-pentafluoropropène-1 (HFO-1225ye), suivi d'une hydrogénation pour produire le composé recherché. La déshydrohalogénation du HFC-236ea en HFO-1225ye est effectuée en phase gazeuse, le produit de la réaction Le document de Sianisi Dario et Al, Annali du Chimica, Societa Chimica, Italia, Rome, vol 55, n° 8-9, 1 janvier 1965, pages 850-861 décrit la préparation du pentafluoropropène à partir d'un hexafluoropropène avec de l'hydroxyde de potassium.

Le document Knunyants et Al, Bulletin of the Academy of Sciences of the USSR, Division of Chemical Sciences, 1 janvier 1960, pages 1312-1317, décrit en quatre étapes la préparation du 2,3,3,3 tétrafluoropropène à partir du perfluoropropène. étant, dans un exemple, envoyé directement au réacteur suivant dans lequel a lieu l'hydrogénation du composé HFO-1225ye en composé HFC-245eb. Il est aussi indiqué dans ce document que le composé HFC-236ea peut être obtenu par hydrogénation de hexafluoropropylène (HFP).

Le document US-P-5679875 décrit la préparation de 1,1,1,2,3-pentafluoropropane par déhydrofluoration catalytique de 1,1,1,2,3,3-hexafluoropropane (HFC-236ea) en 1,2,3,3,3-pentafluoropropène-1 (HFO-1225ye), suivi d'une hydrogénation pour produire le composé recherché. Les réactions sont effectuées en phase gaz. Il est aussi indiqué dans ce document que le composé HFC-236ea peut être obtenu par hydrogénation de hexafluoropropylène (HFP).

Le document WO 2008/030440 décrit la préparation du HFO-1234yf à partir du HFO-1225ye en faisant réagir le HFO-1225ye avec de l'hydrogène en présence d'un catalyseur pour donner le HFC-245eb, puis en faisant réagir le HFC-245eb avec une solution aqueuse basique en présence d'un catalyseur de transfert de phase et un solvant non-aqueux, non-alcoolique.

Le document WO 2008/075017 illustre la réaction de déhydrofluoration du 1,1,1,2,3,3 hexafluoropropane (HFC-236ea) en 1,1,1,2,3 pentafluoropropène (HFO-1225ye) à 150°C en présence d'une solution aqueuse de 50 % en poids de KOH. En l'absence d'un catalyseur de transfert de phase, la conversion au bout de 3 heures et demie est de 57,8 % et la sélectivité en HFO-1225ye est de 52,4 % (essai 1). En présence d'un catalyseur de transfert de phase, cette conversion est atteinte au bout de 2,5 heures seulement et la sélectivité est pratiquement inchangée (essai 4). Comme indiqué au tableau 2 de ce document pour accroître la sélectivité en HFO-1225ye, il est nécessaire d'utiliser un solvant organique.

Il existe un besoin d'un procédé de préparation de 1234yf à partir d'un produit de départ qui soit aisément accessible, et qui conduise au produit recherché avec une sélectivité élevée, de préférence un rendement élevé et avantageusement une productivité élevée.

### RESUME DE L'INVENTION

L'invention fournit donc un procédé de préparation de 2,3,3,3-tétrafluoro-1-propène comprenant les étapes suivantes:
(i) hydrogénation de hexafluoropropylène en 1,1,1,2,3,3-hexafluoropropane;
(ii) déhydrofluoration du 1,1,1,2,3,3-hexafluoropropane obtenu à l'étape précédente en 1,2,3,3,3-pentafluoropropène-1 à l'aide d'un mélange eau et hydroxyde de potassium avec l'hydroxyde de potassium représentant entre 58 et 86 % en poids du mélange et à une température comprise entre 110 et 180°C;
(iii) hydrogénation du 1,2,3,3,3-pentafluoropropène-1 obtenu à l'étape précédente en 1,1,1,2,3-pentafluoropropane;
(iv) déhydrofluoration du 1,1,1,2,3-pentafluoropropane obtenu à l'étape précédente en 2,3,3,3-tétrafluoro-1-propène à l'aide d'un mélange eau et hydroxyde de potassium avec l'hydroxyde de potassium représentant entre 58 et 86 % en poids du mélange et à une température comprise entre 110 et 180°C ;

Selon des modes de réalisation:
- les étapes d'hydrogénation (i) et (iii) sont mises en oeuvre dans le même réacteur, de préférence avec le même catalyseur, une étape de séparation étant éventuellement présente.
- les étapes d'hydrogénation (i) et/ou (iii) sont mises en oeuvre dans un réacteur multiétagé ou dans au moins deux réacteurs en série, une étape de séparation étant éventuellement présente.
- les étapes de déhydrofluoration (ii) et/ou (iv) sont mises en oeuvre dans au moins deux réacteurs en série, une étape de séparation étant éventuellement présente.
- le flux de l'étape (i) comprenant le 1,1,1,2,3,3-hexafluoropropane est envoyé directement vers l'étape (ii) sans séparation des réactifs.
- le flux de l'étape (i) comprenant le 1,1,1,2,3,3-hexafluoropropane est envoyé vers l'étape (ii), après séparation des réactifs non réagis qui sont éventuellement recyclés à l'étape (i).
- le flux de l'étape (ii) comprenant le 1,2,3,3,3-pentafluoropropène-1 est envoyé vers l'étape(iii) après une étape de purification.
- le flux de l'étape (iii) comprenant le 1,1,1,2,3-pentafluoropropane est envoyé directement vers l'étape (iv) sans séparation des réactifs.
- le flux de l'étape (iii) comprenant le 1,1,1,2,3-pentafluoropropane est envoyé vers l'étape (iv), après séparation des réactifs non réagis qui sont éventuellement recyclés à l'étape (iii).

### DESCRIPTION DETAILLEE DE MODES DE REALISATION

L'invention utilise quatre réactions en série, les produits de réaction étant envoyés dans l'étape suivante, éventuellement après avoir subi un traitement par exemple de séparation, si besoin est.

On peut prévoir d'alimenter en partie l'étape suivante par des réactifs ne provenant pas de l'étape précédente.

Dans le procédé, les étapes réactionnelles sont mises en oeuvre en discontinu, semi-continu ou continu. Avantageusement, le procédé selon la présente invention est mis en oeuvre en continu. On obtient ainsi un procédé économique de préparation du composé HFO-1234yf, le produit de départ, HFP, étant disponible facilement sur le marché, à un coût faible.

Les étapes d'hydrogénation sont mises en oeuvre de façon classique pour l'homme du métier. L'homme du métier pourra choisir les conditions opératoires pour que les réactions soient sensiblement quantitatives.

Les catalyseurs susceptibles d'être utilisés dans ces réactions sont ceux connus à cette fin. On peut notamment citer les catalyseurs à base d'un métal du groupe VIII ou rhénium. Ce catalyseur peut être supporté, par exemple sur carbone, carbure de silicium, alumine, fluorure d'aluminium, etc. ou peut ne pas être supporté, comme le nickel de Raney. Comme métal, on peut utiliser du platine ou du palladium, en particulier du palladium, avantageusement supporté sur carbone ou alumine. On peut aussi associer ce métal avec un autre métal comme l'argent, le cuivre, l'or, le tellure, le zinc, le chrome, le molybdène et le thallium. Ces catalyseurs d'hydrogénation sont connus.

Le catalyseur peut être présent sous toute forme appropriée, par exemple sous forme de lit fixe ou fluidisé, de préférence en lit fixe. La direction de flux peut être de haut en bas ou de bas en haut. Le lit de catalyseur peut aussi comprendre une distribution particulière du catalyseur afin de gérer les flux de chaleurs générés par la réaction exothermique. Ainsi, il est possible de prévoir des gradients de densité de charge, de porosité, etc. du catalyseur afin de réguler l'exothermicité de la réaction. Par exemple, on peut prévoir que la première partie du lit comprend moins de catalyseur tandis que la seconde partie en comprend plus.

On peut aussi prévoir des étapes de régénération du catalyseur, de façon connue.

On peut aussi prévoir d'utiliser un gaz de dilution comme l'azote, l'hélium ou l'argon.

Les étapes d'hydrogénation sont exothermiques. La température de réaction peut être contrôlée à l'aide de moyens disposés à cet effet dans le réacteur, si besoin est. La température peut varier de quelques dizaines de degrés au cours de la réaction, la réaction (i) étant plus exothermique que la réaction (iii). Par exemple, la température d'entrée peut varier de 20°C à 120°C ,de préférence entre 50 et 100°C et le gain en température peut varier de 5°C à 100°C.

Le temps de contact (rapport entre le volume de catalyseur et le flux total de la charge) est en général compris entre 0,1 et 100 secondes, de préférence entre 1 et 50 secondes et avantageusement entre 2 et 10 secondes.

La quantité d'hydrogène injectée peut varier dans de grandes mesures. Le ratio H₂/charge peut varier dans de grandes mesures, notamment entre 1 (la quantité stoechiométrique) et 30, notamment entre 1,5 et 20, avantageusement entre 1,1 et 3. Un ratio élevé conduira à une dilution, et donc une meilleure gestion de l'exothermicité de la réaction.

Le flux à l'issue des étapes d'hydrogénation (i) et/ou (iii) peut ou peuvent être envoyé(s) directement à l'étape de déhydrofluoration suivante ou être soumis à une étape de séparation pour séparer les réactifs non réagis (hydrogène, HFP ou HFO-1225ye) avant d'être envoyé à l'étape de déhydrofluoration suivante. Après séparation, les réactifs non réagis peuvent être recyclés.

De préférence, le flux à l'issue des étapes d'hydrogénation (i) et/ou (iii) est ou sont envoyé(s) directement à l'étape de déhydrofluoration suivante.

Les réactions d'hydrogénation de l'étape (i) et/ou (iii) sont, de préférence, sensiblement quantitatives. Elles peuvent être mises en oeuvre dans un réacteur multiétagé ou dans au moins deux réacteurs en série, une étape de séparation étant éventuellement présente.

Les réactions de déhydrofluoration sont mises en oeuvre en faisant réagir du HFC-236ea et/ou HFC-245eb avec un mélange eau et hydroxyde de potassium (KOH), dans lequel l'hydroxyde de potassium est présent entre 58 et 86 % en poids à une température comprise entre 110 et 180°C, de préférence supérieure à 150°C et avantageusement comprise entre 152 et 165°C.

De préférence, l'hydroxyde de potassium est présent entre 60 et 75 % en poids dans le mélange eau-KOH.

Le mélange eau et KOH utilisé peut provenir des hydrates de formule KOH.xH₂O (x étant compris entre 1 et 2). De préférence, les réactions de déhydrofluoration sont mises en oeuvre en présence de ces hydrates d'hydroxydes de potassium à l'état fondu et avantageusement en l'absence de solvant et/ou de catalyseur de transfert de phase.

Le 1,1,1,2,3,3-hexafluoropropane à l'étape (ii) et/ou le 1,1,1,2,3-pentafluoropropane à l'étape (iv) est ou sont converti(s) en général à plus de 90 %, de préférence à plus de 95% et avantageusement à plus de 98 %.

Un gaz diluant (azote, hélium, argon ou hydrogène) peut être utilisé dans la réaction de déhydrofluoration.

La réaction de déhydrofluoration peut être mise en oeuvre dans tout type de réacteur connu de l'homme de l'art. On peut utiliser, un réacteur agité, un mélangeur statique, une colonne réactive, ou tout simplement faire barboter le HFC-236ea et/ou le HFC-245eb dans le mélange eau et KOH dans un récipient. On peut également uitliser au moins deux réacteurs en série.

La quantité de KOH mise en jeu dans les réactions de déhydrofluoration, lorsqu'elles sont mises en oeuvre en discontinu ou semi-continu est telle que le rapport molaire KOH/ HFC-245eb ou HFC-236ea est compris entre 1 et 20.

Au cours des réactions de déhydrofluoration, le fluorure de potassium se forme et pour les réactions conduites en continu, on préfère éliminer en continu ou discontinu tout ou une partie du KF formé du milieu réactionnel. Le fluorure de potassium peut être séparé du milieu réactionnel par filtration.

Au cours des réactions de déhydrofluoration, il se forme de l'eau qui peut également être éliminée en continu ou discontinu de manière à maintenir la teneur de KOH dans le mélange eau-KOH dans l'intervalle précédemment décrit. L'élimination d'eau peut se faire par évaporation.

Le flux à l'issue de l'étape de déhydrofluoration (ii) comprenant le HFO-1225ye peut être envoyé directement à l'étape (iii). De préférence, ce flux est préalablement purifié par exemple par distillation.

Le flux à l'issue de l'étape de déhydrofluoration (iv) comprenant le HFO-1234yf, éventuellement séparé du HFC-245eb, est soumis à une étape de purification, par exemple par distillation.

Il est possible dans le procédé de prévoir que les étapes d'hydrogénation (i) et (iii) sont mises en oeuvre dans le même réacteur, de préférence avec le même catalyseur.

La co-hydrogénation est mise en oeuvre dans un premier réacteur, dont le flux de sortie contient le HFC-236ea et le HFC-245eb. Le flux de sortie peut être séparé et le HFC-236ea est envoyé dans un premier réacteur de déhydrofluoration, tandis que le HFC-245eb est envoyé dans un second réacteur de déhydrofluoration. Le flux de sortie du premier réacteur de déhydrofluoration contient majoritairement du HFO-1225ye et éventuellement du HFC-236ea n'ayant pas réagi. On peut renvoyer le flux de sortie du premier réacteur de déhydrofluoration vers le réacteur d'hydrogénation, produisant ainsi le composé HFC-245eb à partir de ce HFO-1225ye. Le HFC-236ea éventuellement séparé peut être recyclé en tête de ce réacteur de déhydrofluoration.

La pression dans les différentes réactions peut être atmosphérique, ou inférieure ou supérieure à cette pression atmosphérique. La pression peut varier d'une réaction à l'autre, le cas échéant.

L'alimentation en réactifs se fait en général en continu, ou peut être sequencée le cas échéant.

Les réactions sont mises en oeuvre dans un ou plusieurs réacteurs dédiés aux réactions impliquant des halogènes. De tels réacteurs sont connus de l'homme du métier, et peuvent comprendre des revêtements intérieurs à base par exemple de Hastelloy®, Inconel®, Monel® ou de fluoropolymères. Le réacteur peut aussi comprendre des moyens d'échange de chaleur, si besoin.

On rappellera que:
- le taux de conversion est le % du produit de départ ayant réagi (nombre mole de produit départ ayant réagi/nombre mole produit départ introduit) ;
- la sélectivité en produit recherché est le ratio nombre de mole de produit recherché formé/nombre mole produit départ ayant réagi ;
- le rendement en produit recherché est le ratio nombre de mole de produit recherché formé/nombre mole produit départ introduit, le rendement en produit recherché pouvant encore être défini comme le produit de la conversion et de la sélectivité ;
- le temps de contact est l'inverse de la vitesse spatiale VVH (ou WHSV en langue anglaise) ;
- la vitesse spatiale est le rapport entre le débit volumique du flux gazeux total sur le volume du lit catalytique, dans les conditions normales de température et de pression.

### EXEMPLES

Les exemples suivants illustrent l'invention sans la limiter.

### Exemple 1. Hydrogénation de HFP en HFC-236ea.

On utilise un réacteur tubulaire de diamètre interne de 21 mm et de longueur de 1,2 m, à double enveloppe avec circulation d'eau maintenue à 40°C. Le réacteur est chargé de trois lits catalytiques, du type pastille de Pd/supporté sur alumine. Les trois lits catalytiques se différencient par la teneur du Pd supporté et disposés en concentration croissante. Le lit catalytique ayant la teneur en Pd la moins élevée se trouve le plus proche de l'arrivée des réactifs.

Ainsi, le réacteur comprend un lit de 15 cm constitué de catalyseur ayant une teneur de 0,5 % en poids de Pd sur de l'alumine mais dilué avec 5 fois le volume de carbure de silicium, un lit de 10 cm constitué de catalyseur ayant une teneur de 0,5 % en poids de Pd sur de l'alumine non dilué et un lit de 20 cm de catalyseur ayant une teneur de 2,2 % en poids de Pd sur de l'alumine.

Avant de charger les trois lits catalytiques, on a introduit dans le réacteur environ 130 cm³ de Corindon (soit 37 cm). 80 cm³ de Corindon (25 cm) ont également été introduits au-dessus du premier lit catalytique.

Le catalyseur est activé avant sa première mise en service à l'aide d'un flux d'environ 20 l/ h d'hydrogène pendant 12h et à 250°C.

La pression est de 1 bar absolu.

Avec un débit de HFP de 150 g/h (1 mol/h) et d'hydrogène de 33,6 Nl/h (1,5 mol/h) et à conversion totale en HFP, on obtient un rendement de 95,2 % en HFC-236ea.

Pendant la réaction, on a observé une température maximale de 105°C pour le lit catalytique le plus dilué et de 140°C pour les deux autres lits catalytiques.

### Exemple 2. Déhydrofluoration de HFC-236ea en HFO-1225ye.

On utilise deux récipients de volume de 1 litre reliés en série (le flux gazeux issu du premier récipient sert d'alimentation pour le deuxième récipient) et on charge dans chaque récipient 1000 g d'un mélange eau et KOH dans lequel le KOH est présent à 80 % en poids. La température du mélange est maintenue entre 155 et 170°C. On introduit en continu pendant 6 heures 165 g/h du HFC-236ea. Pour une conversion totale en HFC-236ea, on obtient un rendement de 93,9 % en HFO-1225ye.

### Exemple 3. Hydrogénation de HFO-1225ye en HFC-245eb.

On utilise le même réacteur qu'à l'exemple 1 mais avec une charge catalytique comprenant un lit de 23,5cm de catalyseur 0,2 % en poids de Pd sur carbure de silicium (SiC), un lit de 15 cm de catalyseur 0,5 % en poids de Pd supporté sur charbon et un lit de 40cm de catalyseur 2,0 % en poids de Pd sur charbon. La température de l'eau dans la double enveloppe est maintenue à environ 85°C. La pression est de 1 bar absolu.

Pour un débit de HFO-1225ye de 128 g/h et d'hydrogène de 33,6 Nl/h et à conversion totale, on obtient un rendement de 84 % en HFC-245eb.

### Exemple 4. Déhydrofluoration de HFC-245eb en HFO-1234yf.

On utilise un récipient de volume d'un litre contenant 1000 g d'un mélange eau et KOH dans lequel le KOH est présent à 75 % en poids. On introduit en continu pendant 2 heures dans le mélange maintenu à 160°C du HFC-245eb avec un débit de 138/h et on obtient une conversion de 83 % en HFC-245eb pour une sélectivité en HFO-1234yf de 100 %. La pression est de 1 bar.

### Exemple 5

On utilise l'appareillage de l'exemple 3 avec la même composition catalytique sauf que le flux à la sortie du réacteur d'hydrogénation est introduit directement dans le mélange eau et KOH de l'appareillage de l'exemple 2 contenant 850 g de mélange eau et KOH, dans lequel le KOH est présent à 80 % en poids, dans le premier réacteur et 637 g du même mélange dans le deuxième réacteur.

On introduit en continu pendant 5,1 heures dans le réacteur d'hydrogénation 2,79 mol/h d'hydrogène et 1,04 mol/h de HFP et on obtient après passage dans les réacteurs contenant le mélange eau et KOH, une conversion totale de HFP, et un rendement en HFO-1225ye de 92 %.

### Exemple 6

On utilise le même appareillage que l'exemple 5 sauf que l'on introduit en continu pendant 6,4 heures, 1 mole/h de HFO-1225ye non purifié obtenu à l'exemple 5 et 1,5 mole/h d'hydrogène. On obtient pour une conversion de 98 % en HFO-1225ye, un rendement en HFO-1234yf de 96,8 %.

## Revendications

1. Procédé de préparation de 2,3,3,3-tétrafluoro-1-propène comprenant les étapes suivantes:
(i) hydrogénation de hexafluoropropylène en 1,1,1,2,3,3-hexafluoropropane ;
(ii) déhydrofluoration du 1,1,1,2,3,3-hexafluoropropane obtenu à l'étape précédente en 1,2,3,3,3-pentaflucropropène-1 à l'aide d'un mélange eau et hydroxyde de potassium ;
(iii) hydrogénation du 1,2,3,3,3-pentafluoropropène-1 obtenu à l'étape précédente en 1,1,1,2,3-pentafluoropropane ;
(iv) déhydrofluoration du 1,1,1,2,3-pentafluoropropane obtenu à l'étape précédente en 2,3,3,3-tétrafluoro-1-propène à l'aide d'un mélange eau et hydroxyde de potassium **caractérisé en ce que** la déhydrofluoration à l'étape (ii) et (iv) est effectuée à une température comprise entre 110 et 180°C et l'hydroxyde de potassium représente entre 58 et 86 % en poids du mélange.

2. Procédé selon la revendication 1, dans lequel l'hydroxyde de potassium représente entre 60 et 75 % en poids du mélange eau-hydroxyde de potassium de l'étape (ii) et/ou (iv).

3. Procédé selon la revendication 1 ou 2, dans lequel l'étape (ii) et/ou l'étape (iv) est ou sont mise(s) en oeuvre à une température supérieure à 150°C, de préférence comprise entre 152 et 165°C.

4. Procédé selon l'une quelconque des revendications précédentes **caractérisé en ce que** le flux issu de l'étape (i) et/ou l'étape (iii) est ou sont envoyé(s) directement à l'étape suivante.

5. Procédé selon l'une quelconque des revendications précédentes **caractérisé en ce que** le flux issu de l'étape (ii) est soumis à une étape de purification avant d'être envoyé à l'étape (iii).

6. Procédé selon l'une quelconque des revendications précédentes **caractérisé en ce qu'**il est mis en oeuvre en continu.

7. Procédé selon l'une quelconque des revendications précédentes **caractérisé en ce que** l'étape (ii) et/ ou l'étape (iv) est ou sont mis en oeuvre dans au moins deux réacteurs en série.

8. Procédé selon l'une quelconque des revendications précédentes **caractérisé en ce que** l'étape (i) et/ou l'étape (iii) est ou sont mis en oeuvre dans un réacteur multiétagé ou dans au moins deux réacteurs en série.

## Patentansprüche

1. Verfahren zur Herstellung von 2,3,3,3-Tetrafluor-1-propen, das folgende Schritte umfasst:
(i) Hydrierung von Hexafluorpropylen zu 1,1,1,2,3,3-Hexafluorpropan;
(ii) Dehydrofluorierung des im vorhergehenden Schritt erhaltenen 1,1,1,2,3,3-Hexafluorpropans zu 1,2,3,3,3-Pentafluor-1-propen mit Hilfe einer Mischung von Wasser und Kaliumhydroxid;
(iii) Hydrierung des im vorhergehenden Schritt erhaltenen 1,2,3,3,3-Pentafluor-1-propens zu 1,1,1,2,3-Pentafluorpropan;
(iv) Dehydrofluorierung des im vorhergehenden Schritt erhaltenen 1,1,1,2,3-Pentafluorpropans zu 2,3,3,3-Tetrafluor-1-propen mit Hilfe einer Mischung von Wasser und Kaliumhydroxid, **dadurch gekennzeichnet, dass** die Dehydrofluorierung in Schritt (ii) und Schritt (iv) bei einer Temperatur zwischen 110 und 180°C durchgeführt wird und das Kaliumhydroxid zwischen 58 und 86 Gew.-% der Mischung ausmacht.

2. Verfahren nach Anspruch 1, bei dem das Kaliumhydroxid zwischen 60 und 75 Gew.-% der Mischung von Wasser und Kaliumhydroxid aus Schritt (ii) und/oder (iv) ausmacht.

3. Verfahren nach Anspruch 1 oder 2, bei dem Schritt (ii) und/oder Schritt (iv) bei einer Temperatur von mehr als 150°C, vorzugsweise zwischen 152 und 165°C, durchgeführt wird bzw. werden.

4. Verfahren nach einem der vorhergehenden Ansprüche, **dadurch gekennzeichnet, dass** der Strom aus Schritt (i) und/oder Schritt (iii) direkt dem folgenden Schritt unterworfen wird.

5. Verfahren nach einem der vorhergehenden Ansprüche, **dadurch gekennzeichnet, dass** der Strom aus Schritt (ii) vor der Zuführung zu Schritt (iii) einem Reinigungsschritt unterworfen wird.

6. Verfahren nach einem der vorhergehenden Ansprüche, **dadurch gekennzeichnet, dass** es kontinuierlich durchgeführt wird.

7. Verfahren nach einem der vorhergehenden Ansprüche, **dadurch gekennzeichnet, dass** Schritt (ii) und/oder Schritt (iv) in mindestens zwei Reaktoren in Reihe durchgeführt wird bzw. werden.

8. Verfahren nach einem der vorhergehenden Ansprüche, **dadurch gekennzeichnet, dass** Schritt (i) und/oder Schritt (iii) in einem mehrstufigen Reaktor oder in mindestens zwei Reaktoren in Reihe durchgeführt wird bzw. werden.

## Claims

1. Process for the preparation of 2,3,3,3-tetrafluoro-1-propene which comprises the following stages:
(i) hydrogenation of hexafluoropropylene to give 1,1,1,2,3,3-hexafluoropropane;
(ii) dehydrofluorination of the 1,1,1,2,3,3-hexafluoropropane obtained in the preceding stage to give 1,2,3,3,3-pentafluoro-1-propene using a water and potassium hydroxide mixture;
(iii) hydrogenation of the 1,2,3,3,3-pentafluoro-1-propene obtained in the preceding stage to give 1,1,1,2,3-pentafluoropropane;
(iv) dehydrofluorination of the 1,1,1,2,3-pentafluoropropane obtained in the preceding stage to give 2,3,3,3-tetrafluoro-1-propene using a water and potassium hydroxide mixture, **characterized in that** the dehydrofluorination in stage (ii) and (iv) is carried out at a temperature of between 110 and 180°C and the potassium hydroxide represents between 58 and 86% by weight of the mixture.

2. Process according to Claim 1, in which the potassium hydroxide represents between 60 and 75% by weight of the water-potassium hydroxide mixture of stage (ii) and/or (iv).

3. Process according to Claim 1 or 2, in which stage (ii) and/or stage (iv) is or are carried out at a temperature of greater than 150°C, preferably of between 152 and 165°C.

4. Process according to any one of the preceding claims, **characterized in that** the stream resulting from stage (i) and/or stage (iii) is or are conveyed directly to the following stage.

5. Process according to any one of the preceding claims, **characterized in that** the stream resulting from stage (ii) is subjected to a purification stage before being conveyed to stage (iii).

6. Process according to any one of the preceding claims, **characterized in that** it is carried out continuously.

7. Process according to any one of the preceding claims, **characterized in that** stage (ii) and/or stage (iv) is or are carried out in at least two reactors in series.

8. Process according to any one of the preceding claims, **characterized in that** stage (i) and/or stage (iii) is or are carried out in a multistage reactor or in at least two reactors in series.
